# EUROPÄISCHE PATENTSCHRIFT

(11) **EP 0 568 879 B1**
(45) Veröffentlichungstag und Bekanntmachung des Hinweises auf die Patenterteilung: **17.07.1996**
(21) Anmeldenummer: 93106649.2
(22) Anmeldetag: 23.04.1993
(51) Int. Cl.: C07C 45/46, C07C 49/67, C07C 49/675, C07C 49/755, C07C 49/617, C07J 1/00

(54) **Verfahren zur Herstellung substituierter Indanone und ihre Verwendung**
Process for the preparation of substituted indanones and their use
Procédé pour la préparation d'indanones substituées et leur utilisation

(30) Priorität: 28.04.1992 DE 4213940
(43) Veröffentlichungstag der Anmeldung: 10.11.1993
(73) Patentinhaber: HOECHST AKTIENGESELLSCHAFT, 65926 Frankfurt am Main (DE)
(72) Erfinder: Weisse, Laurent, Dr., W-6370 Oberursel (DE); Rohrmann, Jürgen, Dr., W-6233 Kelkheim (Ts.) (DE); Küber, Frank, Dr., W-6370 Oberursel (DE); Strutz, Heinz, Dr., W-6390 Usingen (DE)

(56) Entgegenhaltungen:
- EP-A- 0 203 276
- US-A- 1 754 031
- US-A- 4 322 414
- JOURNAL OF THE AMERICAN CHEMICAL SOCIETY. Bd. 72, 1950, GASTON, PA US Seiten 3286 - 3287 R.T. HART ET AL. 'Acylation - Alkylation Studies. I.'
- JOURNAL OF THE AMERICAN CHEMICAL SOCIETY. Bd. 61, 1939, GASTON, PA US Seiten 1272-1281 L.F. FIESER ET AL.I 'Inter- and Intramolecular Acylations with Hydrogen Fluoride.'
- 1272 - 1281 L.F. FIESER ET AL.I 'Inter- and Intramolecular Acylations with Hydrogen Fluoride.'
- Jerry March, Advanced Organic Chemistry, third Edition, John Wiley, S.484-485

## Beschreibung

Die vorliegende Erfindung betrifft ein technisch einfaches Verfahren zur Herstellung von substituierten 1-Indanonen.

Verbindungen dieses Typs sind wichtige Zwischenprodukte bei der Herstellung von Metallocen-Komplexen, da sich 1-Indanone leicht in die entsprechenden Indene überführen lassen. Indene werden als Ligandsystem zum Aufbau von Metallocen-Komplexen verwendet (EP-A 336 128). Insbesondere die entsprechenden verbrückten, chiralen Zirkon-Derivate besitzen als hochaktive Katalysatoren bei der Olefinpolymerisation große Bedeutung (vgl. EP-A 129 368; EP-A 321 852). Durch Variation des Ligandsystems, z.B. durch Substitution, können die Katalysatoreigenschaften gezielt beeinflußt werden. Hierdurch ist es möglich, die Polymerausbeute, die Molmasse, die Taktizität oder den Schmelzpunkt der Polymere im gewünschten Maß zu verändern (New J. Chem. 14 (1990) 499; Organomet. 9 (1990) 3098; Angew. Chem. 102 (1990) 339; EP-A 316 155; EP-A 351 392).

Weiterhin besitzen substituierte 1-Indanone technische Bedeutung als Riechstoffe (EP-A 162 465) und als wertvolle Zwischenprodukte bei der Herstellung von pharmazeutischen Produkten oder anderen bioaktiven Verbindungen (EP-A 421 759; J. Med. Chem. 25 (1990) 765).

In der Literatur sind mehrere Verfahren zur Herstellung von substituierten 1-Indanonen beschrieben.

1-Indanone, die am Sechsring Substituenten tragen, können ausgehend von den entsprechend substituierten Aromaten durch Ankondensieren des Fünfrings in 2-bis 6-stufigen Synthesen hergestellt werden (J. Org. Chem., 55 (1990) 247; Bull. Soc. Chim. Fr. 6 (1969) 1981).

Herstellungsverfahren für 1-Indanone, die am Fünfring oder an beiden Ringen Substituenten tragen sind ebenfalls bekannt (J. Org. Chem. 46 (1981) 3758; J. Org. Chem. 23 (1958) 1441).

Diese Methoden haben den Nachteil, daß sie in der Regel mehrstufig sind und nur schlechte Gesamtausbeuten an den gewünschten Produkten liefern. Viele der Synthesen sind nicht allgemein anwendbar, sondern auf spezielle Derivate beschränkt. Bei anderen sind wiederum die Ausgangsmaterialien schlecht zugänglich oder sehr teuer. Gewisse Substitutionsmuster am Aromaten sind ebenfalls nach diesen Methoden nicht realisierbar. Die wenigen bekannten einstufigen Synthesen haben den Nachteil, daß sie auf spezielle Derivate beschränkt sind und schlechte Ausbeuten liefern, so daß technisch aufwendige Reinigungsoperationen der Produkte erforderlich sind. Die meisten dieser Reaktionen werden mit Hilfe von Friedel-Crafts-Katalysatoren, wie z.B. AlCl₃, durchgeführt, die im Überschuß eingesetzt werden. Diese Reaktionen erfordern technisch aufwendige Aufarbeitungsschritte, die mit einem großen Salzanfall verbunden sind.

Bekannt sind auch Herstellungsverfahren von substituierten Indanonen durch Umsetzung von Aromaten wie Xylol oder Acenaphthen mit wäßriger Methacrylsäure, Crotonsäure oder Zimtsäure in einem großen Überschuß von flüssigem Fluorwasserstoff (J. Am. Chem. Soc. 61 (1939) 1272; J. Am. Chem. Soc. 72 (1950) 3287). Die Ausbeuten liegen zwischen 62 % und 81 %. Diese Methode hat den Nachteil, daß durch vorhandenes oder gebildetes Wasser erhebliche Korrosionsprobleme verursacht werden. Eine Recyclisierung des Fluorwasserstoffs ist durch die Anwesenheit von Wasser ebenfalls nicht möglich. Die Flußsäure muß neutralisiert werden, wodurch eine große Menge an schwer entsorgbarem Salz anfällt. Außerdem müssen die Produkte aufgrund der niedrigen Ausbeuten noch gereinigt werden.

Es bestand somit die Aufgabe, ein Verfahren zur Herstellung der obengenannten Indanone zu finden, das die aus dem Stand der Technik bekannten Nachteile vermeidet.

Es wurde gefunden, daß Aromaten der nachstehenden Formel I mit käuflichen Carbonsäureanhydriden der Formel II oder Carbonsäurefluoriden der Formel III in flüssigem Fluorwasserstoff nahezu quantitativ zu Indanonen der Formel IV/IVa reagieren.

Die vorliegende Erfindung betrifft daher ein Verfahren zur Herstellung einer Verbindung der Formel IV oder deren Isomer der Formel IVa 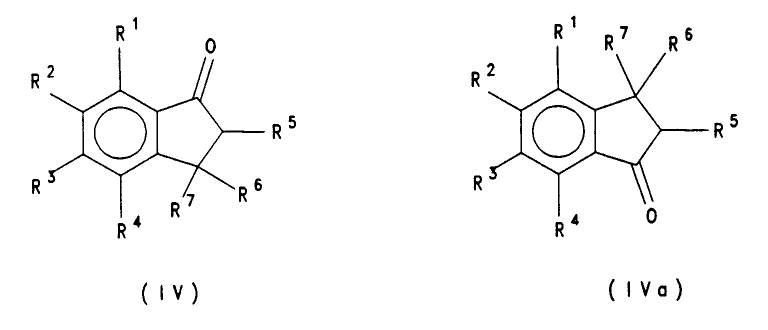
worin
R¹, R², R³, R⁴, R⁵, R⁶ und R⁷ gleich oder verschieden sind und Wasserstoff, (C₁-C₂₀)-Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest -SiR⁸₃, wobei R⁸ für (C₁-C₁₀)Alkyl steht, ein Halogenatom oder einen heteroaromatischen Rest mit 5 oder 6 Ringgliedern, der ein oder mehrere Heteroatome enthalten kann, bedeuten, oder benachbarte Reste R¹-R⁴ mit den sie verbindenden Atomen einen oder mehrere substituierte oder unsubstituierte Ringe bilden, dadurch gekennzeichnet, daß man eine Verbindung der Formel I 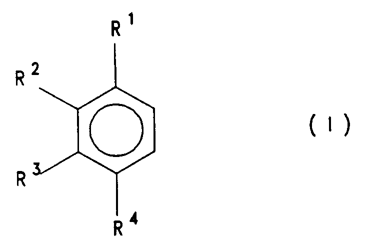
mit einer Verbindung der Formel II 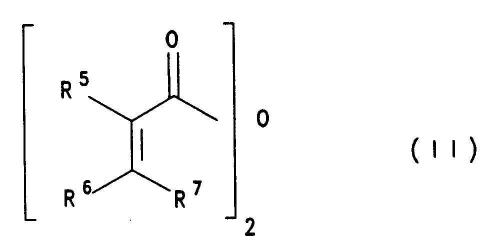
oder mit einer Verbindung der Formel (III) 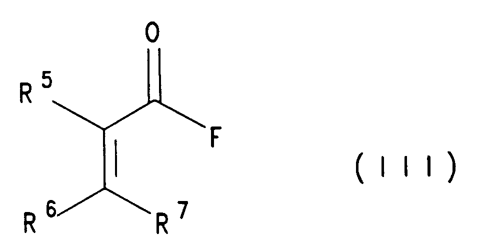
worin R¹ - R⁷ die genannten Bedeutungen besitzen, in flüssigem, wasserfreiem Fluorwasserstoff umsetzt.

Dabei steht Alkyl für geradkettiges oder verzweigtes Alkyl. Halogen bedeutet Fluor, Chlor, Brom oder Jod, insbesondere Fluor oder Chlor. Beispiele für heteroaromatische Reste sind Thienyl, Furyl oder Pyridyl.

Die von benachbarten Resten R¹-R⁴ gebildeten Ringe können durch Substituenten in der Bedeutung von R¹-R⁷, einschließlich der dafür genannten Vorzugsbereiche, substituiert sein.

In den Formeln IV bzw. IVa gilt bevorzugt, daß R¹, R², R³, R⁴ gleich oder verschieden sind und Wasserstoff, (C₁-C₁₀)Alkyl, (C₆-C₁₄)Aryl, (C₁-C₄)Alkoxy, (C₂-C₆)Alkenyl, (C₁-C₆)Fluoralkyl oder ein Halogenatom bedeuten oder die Reste R¹ und R², R² und R³ oder R³ und R⁴ mit den sie verbindenden Atomen einen substituierten oder unsubstituierten fünf- oder sechsgliedrigen Ring bilden, R⁵ (C₁-C₁₀)Alkyl und R⁶ und R⁷ Wasserstoff bedeuten.

Insbesondere gilt, daß R¹, R², R³ und R⁴ gleich oder verschieden sind und Wasserstoff oder (C₁-C₁₀)Alkyl bedeuten, oder die Reste R¹ und R², R² und R³ oder R³ und R⁴ mit den sie verbindenden Atomen einen substituierten oder unsubstituierten sechsgliedrigen, gesättigten oder ungesättigten Carbocyclus bilden, R⁵ für Methyl steht, und R⁶ und R⁷ Wasserstoff bedeuten.

Der von benachbarten Substituenten R¹ - R⁴ gebildete gesättigte oder ungesättigte Fünf- oder Sechsring (Carbocyclus) kann zusätzlich Substituenten, bevorzugt
(C₁-C₁₀)Alkyl, tragen.

Die Indanone können in Abhängigkeit des Substitutionsmusters am Aromaten in Form zweier Konstitutionsisomere der Formel IV bzw. IVa anfallen. Je nach Verwendungszweck können diese in reiner Form oder als Gemisch weiter umgesetzt werden. Bei der Herstellung von Metallocen-Komplexen kann die Isomerenmischung eingesetzt werden.

Bevorzugt werden die Indanone IV/IVa durch Umsetzung von Aromaten der Formel I mit Anhydriden der Formel II hergestellt.

Die Ausgangsverbindungen der Formel I sind bekannt und im Handel erhältlich. Die Ausgangsverbindungen der Formel II sind im Handel erhältlich oder können nach literaturbekannten Methoden hergestellt werden (vgl. z.B. Advanced Organic Chemistry, 1983, 369).

Die Carbonsäurefluoride der Formel III können aus den bekannten Carbonsäurechloriden oder Carbonsäureanhydriden (Formel II) in literaturbekannter Weise durch Umsetzung mit HF hergestellt werden (vergl. z.B. Advanced Organic Chemistry, 1983, 399).

Bei der Herstellung der Verbindungen IV/IVa kann dem Fluorwasserstoff zusätzliches Lösemittel zugesetzt werden, bevorzugt wird jedoch in reinem, wasserfreiem Fluorwasserstoff gearbeitet.

Die Molverhältnisse der Ausgangsverbindungen einschließlich des Fluorwasserstoffs können innerhalb weiter Grenzen schwanken. Bevorzugt ist das Molverhältnis von Verbindung I : II (bzw. III): HF = 1 : 0,5-2,0 : 5-100; insbesondere
1 : 0,9-1,2 : 20-50. D.h. es wird in einem Überschuß von Fluorwasserstoff gearbeitet.

Die Reaktionstemperatur beträgt bevorzugt -30°C bis 130°C, insbesondere 0°C bis 80°C.

Die Reaktionszeiten schwanken in der Regel zwischen 30 min und 50 h, vorzugsweise zwischen 1 h und 24 h.

Bevorzugt wird die Reaktion in einem Druckbereich von 1-15 atm durchgeführt.

Bevorzugt wird eine Mischung der Verbindungen I und II (bzw. III) vorgelegt und der Fluorwasserstoff zudosiert. Auch die umgekehrte Zugabefolge ist möglich.

Nach Ende der Reaktion kann der Fluorwasserstoff abdestilliert und nahezu quantitativ ohne nennenswerte Verunreinigungen zurückgewonnen werden.

Die Indanone der Formel IV bzw. IVa können durch Waschen mit Na₂CO₃-, NaHCO₃- oder KOH-Lösung und Wasser von Säureanteilen befreit und mit den üblichen Trockenmitteln wie Na₂SO₄, MgSO₄ oder Molekularsieben getrocknet werden. Da die Umsetzungen in der Regel nahezu quantitativ sind, kann in den meisten Fällen auf eine weitere Reinigung verzichtet werden. Oft empfiehlt sich jedoch eine Filtration über Kieselgel, Aluminiumoxid oder Filterhilfsmitteln, wie z.B. Celite. Falls notwendig, kann die weitere Reinigung durch Destillation, Säulenchromatographie oder Kristallisation erfolgen. Falls erforderlich können die Konstitutionsisomeren IV und IVa durch Säulenchromatographie an Kieselgel oder Aluminiumoxid voneinander getrennt werden.

Das erfindungsgemäße Verfahren zeichnet sich besonders dadurch aus, daß unterschiedlich substituierte 1-Indanone in einer einfachen und kurzen Synthese (Einstufenprozeß) sehr selektiv und in nahezu quantitativer Ausbeute erhalten werden können. Eine aufwendige Reinigung der Derivate ist daher im Gegensatz zum Stand der Technik nicht notwendig. Ein weiterer Vorteil ist, daß der als Katalysator dienende Fluorwasserstoff nahezu quantitativ zurückgewonnen und wiederverwendet werden kann, da während der Reaktion kein Wasser gebildet wird. Dies hat den weiteren technisch entscheidenden Vorteil, daß Korrosionsprobleme, verursacht durch wäßrige Flußsäure, vermieden werden. Somit stellt diese Methode ein ökonomisch und ökologisch sehr günstiges Herstellungsverfahren für substituierte 1-Indanone dar. Das Substitutionsmuster am Fünf- und Sechsring kann dabei in einem sehr weiten Bereich variiert werden. Dadurch sind auch neue 1-Indanonderivate zugänglich.

Bevorzugt werden die Indanone IV/IVa zur Herstellung von Metallocenen verwendet, die als hochaktive Katalysatorkomponenten bei der Olefinpolymerisation verwendet werden (vgl. z.B. EP-A 336 128). Dazu werden die Indanone, bevorzugt als Isomerengemisch, zunächst nach literaturbekannten Methoden mit Reduktionsmitteln wie NaBH₄ oder LiAlH₄ zu den entsprechenden Indanolen reduziert und diese anschließend mit Säuren wie Schwefelsäure, Oxalsäure, p-Toluolsulfonsäure oder auch durch Behandlung mit wasserentziehenden Substanzen wie Magnesiumsulfat, Natriumsulfat, Aluminiumoxid, Kieselgel oder Molekularsieben zu den entsprechenden Indenen dehydratisiert (Bull. Soc. Chim. Fr. 11 (1973) 3092; Organomet. 9 (1990) 3098).

Die substituierten Indene können als Doppelbindungsisomere anfallen. Diese können von Nebenprodukten durch Destillation, Säulenchromatographie oder Kristallisation gereinigt werden. Die Isomere können als Gemisch direkt für die Synthese der entsprechenden Metallocen-Komplexe eingesetzt werden.

Die Synthese der Metallocene, ausgehend von Indenen, ist bekannt (AU-A-31478/89; J. Organomet. Chem. 342 (1988) 21; EP-A 284 707).

Die nachfolgenden Beispiele dienen der näheren Erläuterung der Erfindung.

### Beispiel A

### 6,7-Benzo-2-methylindan-1-on (1) und 4,5-Benzo-2-methylindan-1-on (1a)

12,6 g (98 mmol) Naphthalin und 15,8 g (103 mmol) Methacrylsäureanhydrid wurden in einem 250 ml Edelstahlautoklaven mit 100 g (5 mol) wasserfreier HF versetzt und 18 h bei 50°C gerührt. Anschließend wurde der Fluorwasserstoff abdestilliert, der Rückstand in Essigester aufgenommen und mit verdünnter KOH-Lösung neutralisiert. Die abgetrennte wäßrige Phase wurde noch zweimal mit Essigester extrahiert. Die vereinigten organischen Phasen wurden mit MgSO₄ getrocknet und unter vermindertem Druck vom Lösemittel befreit. Man erhielt 19,0 g (99 % d.Th.) eines hellbraunen Öls. Die Selektivitäten zu 1 bzw. 1a betrugen 58 % bzw. 39 %.

¹H-NMR-Spektren (100 MHz, CDCl₃): 1: 9,15 (m,1H), 7,40-8,10 (m,5H), 3,47 (dd,1H), 2,62-2,95 (m,2H), 1,37 (d,3H); 1a: 7,4-8,0 (m,6H), 3,70 (dd,1H), 2,75-3,10 (m,2H), 1,40 (d,3H).
Massenspektrum: 196 M⁺, korrektes Zerfallsmuster.

### Beispiel B

### 6,7-Benzo-2-methylindan-1-on (1) und 4,5-Benzo-2-methylindan-1-on (1a)

In einem 2l Edelstahlautoklaven wurden 120 g (0,94 mol) Naphthalin und 153 ml (1,03 mol) Methacrylsäureanhydrid vorgelegt und bei Raumtemperatur langsam mit 1000 g HF versetzt. Die Mischung wurde langsam auf 60°C erwärmt und 18 h bei dieser Temperatur gehalten. Der Fluorwasserstoff wurde anschließend bei 30-35°C abkondensiert und zurückgewonnen. Der Rückstand wurde in Ethylacetat aufgenommen, zweimal mit Wasser, zweimal mit gesättigter NaHCO₃-Lösung und einmal mit NaCl-Lösung gewaschen. Nach dem Filtrieren über Kieselgel und Abziehen des Lösemittels unter vermindertem Druck erhielt man 180 g (98 % d.Th.) der reinen Isomerenmischung 1/1a. Die Selektivitäten zu 1 bzw. 1a betrugen 60 % bzw. 40 %.

### Beispiel C

### 5,7-Diisopropyl-2-methylindan-1-on (2) bzw. 4,6-Diisopropyl-2-methylindan-1-on (2a)

15,6 g (96 mmol) 1,3-Diisopropylbenzol und 15,8 g (103 mmol) Methacrylsäureanhydrid wurden analog Beispiel A mit HF umgesetzt und aufgearbeitet. Man erhielt 22 g (99 % d.Th.) eines hellbraunen Öls. Die Selektivitäten zu 2 bzw. 2a betrugen 66 % bzw. 30 %.
¹H-NMR-Spektrum (360 MHz, CDCl₃): Isomerenmischung 7,49 (d), 7,36 (d), 7,13 (s), 7,10 (s), 4,15 (sept.), 3,25-3,40 (m), 3,10 (sept), 2,90-3,00 (m), 2,60-2,73 (m), 1,22-1,30 (m). Massenspektrum: 230 M⁺, korrektes Zerfallsmuster.

### Beispiel D

### 5,7-Diisopropyl-2-methylindan-1-on (2) bzw. 4,6-Diisopropyl-2-methylindan-1-on (2a)

15,6 g (96 mmol) 1,3-Diisopropylbenzol und 15,8 g (103 mmol) Methacrylsäureanhydrid wurden analog Beispiel C umgesetzt und aufgearbeitet. Die Rohmischung wurde an 700 g Kieselgel 60 chromatographiert. Mit einem Laufmittelgemisch aus Hexan/Ethylacetat (20:1), dessen Zusammensetzung im Laufe der Chromatographie auf ein Verhältnis von 10:1 verändert wurde, ließen sich zunächst 14,0 g (63 % d.Th.) des Indanons 2 und anschließend 6,2 g (28 % d.Th.) des Indanons 2a eluieren. Die Verbindungen fallen in Form farbloser bis gelblicher Öle an.

¹H-NMR-Spektrum 2 (360 MHz, CDCl₃): 7,13 (s, 1H), 7,10 (s, 1H), 4,15 (sept.,1H), 3,30 (m,1H), 2,95 (sept.,1H); 2,65 (m,2H), 1,23-1,32 (m,15H). ¹H-NMR-Spektrum 2a (360 MHz, CDCl₃): 7,49 (d,1H), 7,36 (d,1H), 3,35 (m,1H), 3,09 (sept.,1H), 2,95 (sept.,1H); 2,70 (m,2H), 1,24-1,33 (m,15H).

### Beispiel E

### 2,5-Dimethylindan-1-on (3) und 2,6-Dimethylindan-1-on (3a)

9,21 g (100 mmol) Toluol und 15,4 g (100 mmol) Methacrylsäureanhydrid wurden in einem 250 ml Edelstahlautoklaven mit 100 g (5 mol) HF versetzt und 4h bei 50°C gerührt. Die Aufarbeitung erfolgte analog Beispiel A. Man erhielt 15,2 g (95 %) des Produktgemisches als hellbraunes Öl. Die Selektivitäten zu 3 bzw. 3a betrugen 85 % bzw. 6 %.
¹H-NMR-Spektrum (100 MHz, DMSO): 7,14-7,59 (m), 3,15-3,50 (m), 2,45-2,80 (m), 2,4 (s), 1,12-1,27 (d). Massenspektrum: 160 M⁺, korrektes Zerfallsmuster.

### Beispiel F

### 5-Isobutyl-2-methylindan-1-on (4)

13,4 g (100 mmol) Isobutylbenzol und 15,4 g (100 mmol) Methacrylsäureanhydrid wurden in einem 250 ml Edelstahlautoklaven mit 100 g (5 mol) HF versetzt und 5 h bei 50°C gerührt. Die Aufarbeitung erfolgte analog Beispiel A. Man erhielt 19,4 g (96 %) des Produktes 4 als bräunliches Öl. Nach dem Filtrieren über Kieselgel mit Essigester erhielt man 18,5 g (92 %) des reinen Indanons 4 als gelbliches Öl.

¹H-NMR-Spektrum (100 MHz, CDCl₃): 7,7 (m), 7,2 (m), 3,35 (q), 2,70 (m), 2,58 (d), 1,95 (q), 1,25 (d), 0,93 (d). Massenspektrum: 202 M⁺, korrektes Zerfallsmuster.

### Beispiel G

### 2,5,7-Trimethyl-1-indanon (5) und 2,4,6-Trimethyl-1-indanon (5a)

10,6 g (100 mmol) m-Xylol (99 %) und 15,4g (100 mmol) Methacrylsäureanhydrid wurden analog Beispiel A mit HF 8 h bei Raumtemperatur umgesetzt und aufgearbeitet. Man erhielt 18 g (≈ 100 %) des Produktes 5 + 5a als braunes Öl. Die Destillation des Rohproduktes bei 80-84°C/0,1 mbar lieferte 16,0 g (92 %) der Isomerenmischung 5 und 5a als farbloses bis leicht gelbliches Öl. Das Molverhältnis von 5 zu 5a ist 1:1.

Massenspektrum: 174 M⁺, korrektes Zerfallsmuster.
¹H-NMR-Spektrum (300 MHz, CDCl₃): 7,38 (s, 1H), 7,22 (s, 1H), 7,07 (s, 1H), 6,89 (s, 1H), 3,18 - 3,32 (m, 2H), 2,46 - 2,74 (m, 7H), 2,35 - 2,38 (2s, 6H), 2,29 (s, 3H), 1,30 (d, 3H), 1,26 (d, 3H).

### Beispiel H

### 2-Methylindan-1-on (6)

7,8 g (100 mmol) Benzol und 15,4 g (100 mmol) Methacrylsäureanhydrid wurden analog Beispiel A mit HF 4 h bei Raumtemperatur umgesetzt und aufgearbeitet. Man erhielt 13,7 g (94 %) des Produktes 6 als braunes Öl. Das Rohprodukt wurde an 200 g Kieselgel 60 chromatographiert. Mit einem Laufmittelgemisch aus Hexan/Methylenchlorid 1:1 ließen sich 12,2 g (84 %) des Indanons 6 als farbloses Öl gewinnen.
¹H-NMR-Spektrum (100 MHz, CDCl₃): 7,5 (m), 3,33 (q), 2,73 (m), 1,30 (d). Massenspektrum: 146 M⁺, korrektes Zerfallsmuster.

### Beispiel J

### 2,4,5,6-Tetramethylindan-1-on (7)

12 g (100 mmol) 1,2,3-Trimethylbenzol und 15,4 g (100 mmol) Methacrylsäureanhydrid wurden analog Beispiel A mit HF 6 h bei Raumtemperatur umgesetzt und aufgearbeitet. Man erhielt 18,0 g (96 %) des Indanons 7 als braunes Öl. Die Destillation des Rohprodukts bei 0,05 Torr/98-104°C lieferte 17,4 g (93 %) der reinen Verbindung 7 als farbloses Öl.
¹H-NMR-Spektrum (100 MHz, CDCl₃): 7,2 (s, 1H), 3,20 (m, 14), 2,4 - 2,8 (m, 11H), 1,25 (d). Massenspektrum: 188 M⁺, korrektes Zerfallsmuster.

### Beispiel K

### 5-Phenyl-2-methylindan-1-on (8)

15,4 g (100 mmol) Biphenyl und 16 g (104 mmol) Methacrylsäureanhydrid wurden analog Beispiel A mit 100 g (5 mol) HF 60 h bei 70 °C umgesetzt. Nach der analog Beispiel A durchführten Aufarbeitung wurden 23 g (8) mit einer Reinheit von 90 % erhalten (93 % d. Th.).

### Beispiel L

### 8-Methyl-4,5,7,8-tetrahydrocyclopenta[e]acenaphthylen-9-on (9)

30,84 g (200 mmol) Acenaphthen und 35 g (228 mmol) Methacrylsäureanhydrid wurden analog Beispiel A mit 50 g (2,5 mol) HF 20 h bei 70 °C umgesetzt. Nach analog Beispiel A durchgeführten Aufarbeitung wurden 44 g (9) isoliert mit einer Reinheit von 92 % (90 % d.Th.).

### Beispiel M

### 2-Methyl-3,9-dihydro-2H-cyclopenta[b]-fluoren-1-on (10) und 2-Methyl-2,10-dihydro-1H-cyclopenta[a]fluoren-3-on (10a)

33,24 g (200 mmol) Fluoren und 35 g (228 mmol) Methacrylsäureanhydrid wurden analog Beispiel A mit 50 g (2,5 mol) HF 25 h bei 70 °C umgesetzt. Nach analog Beispiel A durchgeführter Aufarbeitung wurden 46 g (10) und (10a) isoliert. Die Reinheit von (10) und (10a) war 94% (Ausbeute: 91,5 % d.Th.), wobei das Molverhältnis von (10) zu (10a) 2:1 betrug.

### Beispiel N

### 16-Methyl-6,7,15,16-tetrahydrocyclopenta[a]phenanthren-17-on (11) und 9-Methyl-5,6,9,10-tetrahydro-cyclopenta[b]phenanthren-8-on (11a)

18 g (100 mmol) 9,10-Dihydrophenanthren und 16 g (104 mmol) Methacrylsäureanhydrid wurden analog Beispiel A mit 90 g (4,5 mol) HF 3 h bei 70 °C umgesetzt. Nach analog Beispiel A durchgeführten Aufarbeitung wurden 24,7 g (11) und (11a) isoliert. Die Reinheit von 11 und 11a lag bei 92 % (Ausbeute: 91 % d.Th.), wobei das Molverhältnis von 11 zu 11a 6:4 betrug.

### Beispiel O

### 5-Methoxy-2-methylindan-1-on (12)

10,8 g (100 mmol) Anisol und 16 g (104 mmol) Methacrylsäureanhydrid wurden analog Beispiel A mit 100 g (5 mol) HF 3h bei 70 °C umgesetzt. Nach der analog Beispiel A durchführten Aufarbeitung wurden 17 g (12) mit einer Reinheit von 68 % erhalten (Ausbeute: 65 % d.Th.).

### Beispiel P

### 5,6-Dimethoxy-2-methylindan-2-on (13)

13,82 g (100 mmol) Veratrol und 16 g (104 mmol) Methacrylsäureanhydrid wurden analog Beispiel A mit 100 g (5 mol) HF 18 h bei 30 °C umgesetzt. Nach der analog Beispiel A durchführten Aufarbeitung wurden 20,3 g (13) mit einer Reinheit von 96 % erhalten (Ausbeute: 93,5 % d.Th.).

## Patentansprüche

1. Verfahren zur Herstellung einer Verbindung der Formel IV oder deren Isomer der Formel IVa 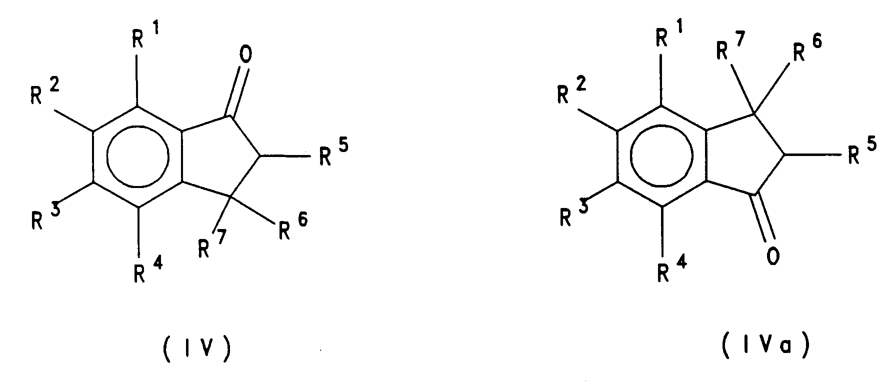 worin
R¹, R², R³, R⁴, R⁵, R⁶ und R⁷ gleich oder verschieden sind und Wasserstoff, (C₁-C₂₀)-Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest
-SiR⁸₃, wobei R⁸ für (C₁-C₁₀)Alkyl steht, ein Halogenatom oder einen heteroaromatischen Rest mit 5 oder 6 Ringgliedern, der ein oder mehrere Heteroatome enthalten kann, bedeuten, oder benachbarte Reste R¹-R⁴ mit den sie verbindenden Atomen einen oder mehrere substituierte oder unsubstituierte Ringe bilden, dadurch gekennzeichnet, daß man eine Verbindung der Formel I 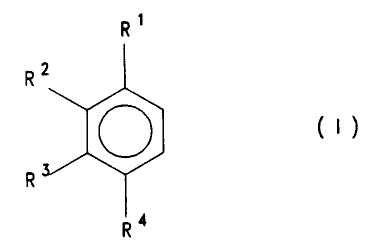 mit einer Verbindung der Formel II 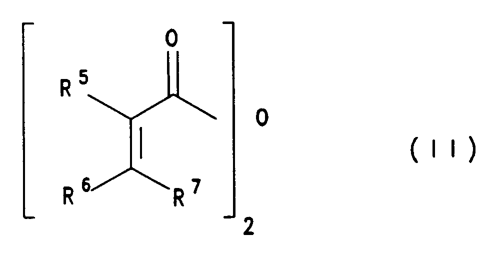 oder mit einer Verbindung der Formel (III) 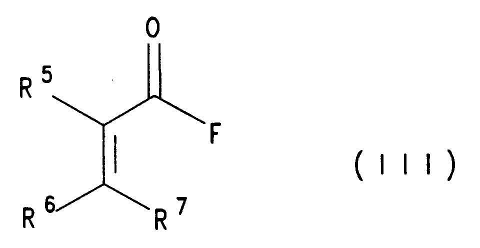 worin R¹ - R⁷ die genannten Bedeutungen besitzen, in flüssigem, wasserfreiem Fluorwasserstoff umsetzt.

2. Verfahren gemäß Anspruch 1, dadurch gekennzeichnet, daß in den Formeln IV und IVa R¹, R², R³, R⁴ gleich oder verschieden sind und Wasserstoff,
(C₁-C₁₀)Alkyl, (C₁-C₄)Alkoxy, (C₂-C₆)Alkenyl, (C₁-C₆)Fluoralkyl oder ein Halogenatom bedeuten oder die Reste R¹ und R², R² und R³ oder R³ und R⁴ mit den sie verbindenden Atomen einen substituierten oder unsubstituierten fünf- oder sechsgliedrigen Ring bilden, R⁵ (C₁-C₁₀)Alkyl und R⁶ und R⁷ Wasserstoff bedeuten.

3. Verfahren gemäß Anspruch 1 oder 2, dadurch gekennzeichnet, daß in den Formeln IV und IVa R¹, R², R³ und R⁴ gleich oder verschieden sind und Wasserstoff oder (C₁-C₁₀)Alkyl bedeuten, oder die Reste R¹ und R², R² und R³ oder R³ und R⁴ mit den sie verbindenden Atomen einen substituierten oder unsubstituierten sechsgliedrigen, gesättigten oder ungesättigten Carbocyclus bilden, R⁵ für Methyl steht, und R⁶ und R⁷ Wasserstoff bedeuten.

4. Verfahren gemäß einem oder mehreren der Ansprüche 1 bis 3, dadurch gekennzeichnet, daß das molare Verhältnis von Verbindung I : Verbindung II oder Verbindung III: Fluorwasserstoff 1 : 0,5-2,0 : 5-100 beträgt.

5. Verfahren gemäß einem oder mehreren der Ansprüche 1 bis 4, dadurch gekennzeichnet, daß eine Verbindung der Formel I mit einer Verbindung der Formel II umgesetzt wird.

## Claims

1. A process for the preparation of a compound of the formula IV or the isomer thereof of the formula IVa 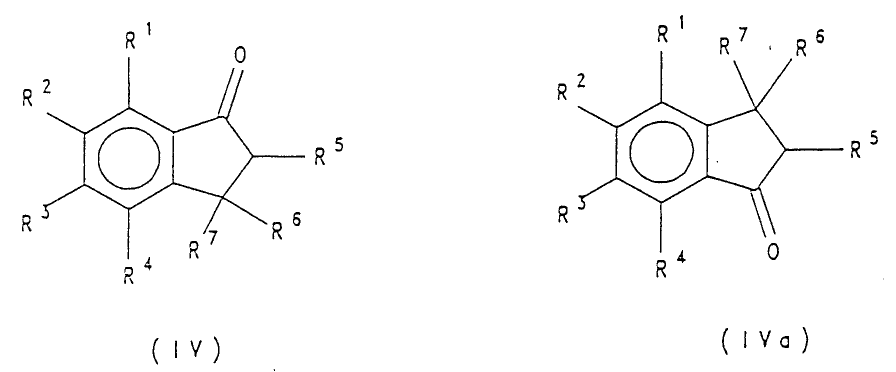 in which
R¹, R², R³, R⁴, R⁵, R⁶ and R⁷ are identical or different and are hydrogen, (C₁-C₂₀)-alkyl, (C₆-C₁₄)aryl, (C₁-C₁₀)alkoxy, (C₂-C₁₀)alkenyl, (C₇-C₂₀)arylalkyl, (C₇-C₂₀)alkylaryl, (C₆-C₁₀)aryloxy, (C₁-C₁₀)fluoroalkyl, (C₆-C₁₀)haloaryl, (C₂-C₁₀)alkynyl, an -SiR⁸₃ radical in which R⁸ is (C₁-C₁₀)alkyl, or are a halogen atom or a heteroaromatic radical having 5 or 6 ring members which may contain one or more heteroatoms, or the adjacent radicals R¹-R⁴, together with the atoms connecting them, form one or more substituted or unsubstituted rings, which comprises reacting a compound of the formula I 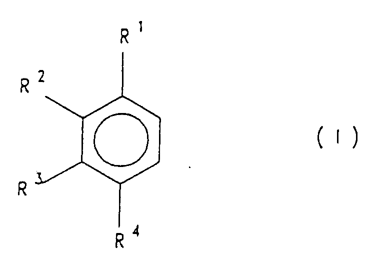 with a compound of the formula II 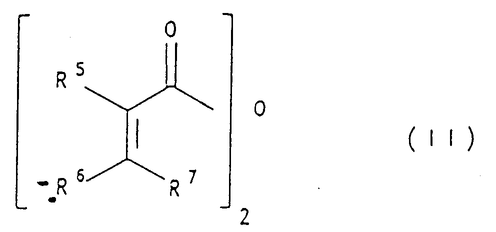 or with a compound of the formula (III) 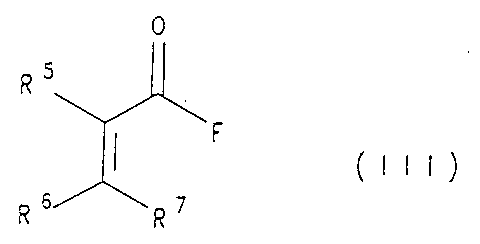 in which R¹-R⁷ are as defined above, in liquid, anhydrous hydrogen fluoride.

2. The process as claimed in claim 1, wherein, in the formulae IV and IVa, R¹, R², R³ and R⁴ are identical or different and are hydrogen, (C₁-C₁₀)alkyl, (C₁-C₄)alkoxy, (C₂-C₆)alkenyl, (C₁-C₆)fluoroalkyl or a halogen atom, or the radicals R¹ and R², R² and R³ or R³ and R⁴, together with the atoms connecting them, form a substituted or unsubstituted, five- or six-membered ring, R⁵ is (C₁-C₁₀)alkyl and R⁶ and R⁷ are hydrogen.

3. The process as claimed in claim 1 or 2, wherein, in the formulae IV and IVa, R¹, R², R³ and R⁴ are identical or different and are hydrogen or (C₁-C₁₀)alkyl, or the radicals R¹ and R², R² and R³ or R³ and R⁴, together with the atoms connecting them, form a substituted or unsubstituted, six-membered, saturated or unsaturated carbocyclic ring, R⁵ is methyl, and R⁶ and R⁷ are hydrogen.

4. The process as claimed in one or more of claims 1 to 3, wherein the molar ratio between compound I: compound II or compound III: hydrogen fluoride is 1:0.5-2.0:5-100.

5. The process as claimed in one or more of claims 1 to 4, wherein a compound of the formula I is reacted with a compound of the formula II.

## Revendications

1. Procédé pour préparer un composé de Formule IV ou son isomère de Formule IVa 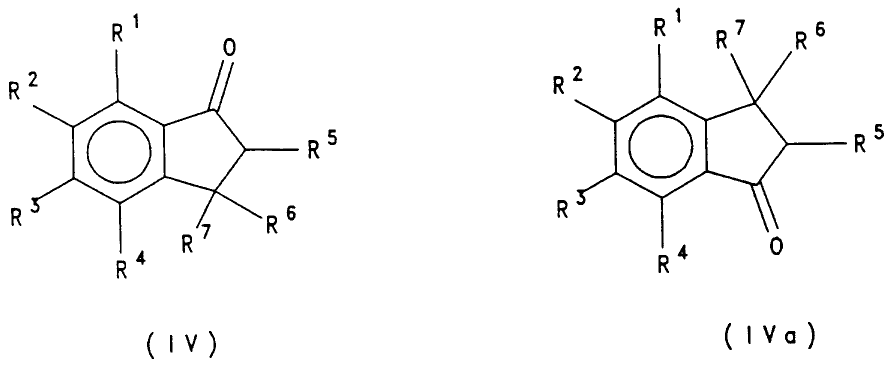 dans lesquelles :
R¹, R², R³, R⁴, R⁵, R⁶ et R⁷ sont identiques ou différents et représentent chacun un hydrogène, un groupe alkyle en C₁-C₂₀, aryle en C₆-C₁₄, alcoxy en C₁-C₁₀, alcényle en C₂-C₁₀, arylalkyle en C₇-C₂₀, alkylaryle en C₇-C₂₀, aryloxy en C₆-C₁₀, fluoralkyle en C₁-C₁₀, halogénaryle en C₆-C₁₀, alcynyleen C₂-C₁₀, un radical -SiR⁸₃, où R⁸ est un groupe alkyle en C₁-C₁₀, ou encore un atome d'halogène ou un résidu hétéroaromatique ayant 5 ou 6 chaînons dans le cycle, qui peut contenir un ou plusieurs hétéroatomes, ou encore des résidus voisins R¹-R⁴ forment, avec les atomes qui les relient, un ou plusieurs noyaux substitués ou non substitués, caractérisé en ce qu'on fait réagir dans du fluorure d'hydrogène liquide anhydre un composé de Formule I 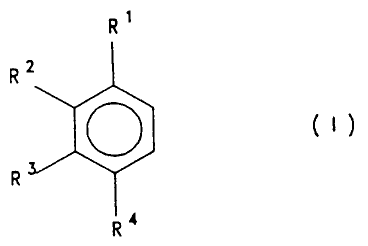 avec un composé de Formule II 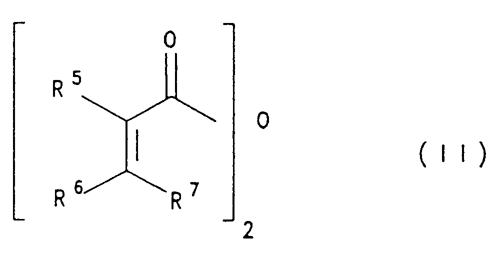 ou avec un composé de Formule III 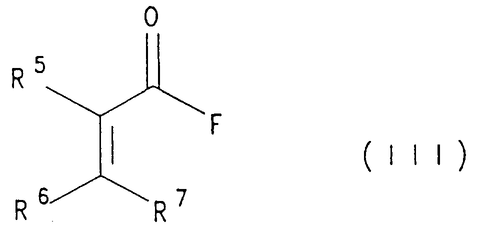 dans lesquelles R¹ à R⁷ ont les significations données ci-dessus.

2. Procédé selon la revendication 1, caractérisé en ce que, dans les Formules IV et IVa R¹, R², R³, R⁴ sont identiques ou différents et représentent chacun un hydrogène ou un groupe alklyle en C₁-C₁₀, alcoxy en C₁-C₄, fluoralkyle en C₁-C₆ ou un atome d'halogène, ou encore les radicaux R¹ et R², R² et R³, ou R³ et R⁴, forment avec les atomes qui les relient un noyau à cinq ou six chaînons, substitué ou non substitué, R⁵ est un groupe alkyle en C₁-C₁₀, et R⁶ et R⁷ sont chacun un hydrogène.

3. Procédé selon la revendication 1 ou 2, caractérisé en ce que, dans les Formules IV et IVa, R¹, R², R³ et R⁴ sont identiques ou différents et représentent chacun un hydrogène ou un groupe alkyle en C₁-C₁₀, ou encore les radicaux R¹ et R², R² et R³ ou R³ et R⁴ forment, avec les atomes qui les relient, un radical carbocyclique saturé ou insaturé, à six chaînons, substitué ou non substitué, R⁵ est le groupe méthyle, et R⁶ et R⁷ sont chacun hydrogène.

4. Procédé selon l'une ou plusieurs des revendications 1 à 3, caractérisé en ce que la proportion en moles composé I:composé II ou composé III:fluorure d'hydrogène est de 1:0,5-2,0:5-100.

5. Procédé selon l'une ou plusieurs des revendications 1 à 4, caractérisé en ce qu'on fait réagir un composé de Formule I avec un composé de Formule II.
